# EUROPEAN PATENT SPECIFICATION

(11) **EP 0 870 834 B1**
(45) Date of publication and mention of the grant of the patent: **30.06.2004**
(21) Application number: 98106586.5
(22) Date of filing: 09.04.1998
(51) Int. Cl.: C12N 15/60, C12N 9/88, C12N 1/21

(54) **Pectic acid lyase**
Pektinsäure-Lyase
Lyase d'acide pectique

(30) Priority: 09.04.1997 JP 9114297; 08.09.1997 JP 24273597
(43) Date of publication of application: 14.10.1998
(73) Proprietor: KAO CORPORATION, Chuo-ku, Tokyo (JP)
(72) Inventor: Hatada, Yuji, Ichikai-machi, Haga-gun, Tochigi (JP); Koike, Kenzo, Sumida-ku, Toyko (JP); Yoshimatsu, Tadashi, Ichikai-machi, Haga-gun, Tochigi (JP); Suzumatsu, Atsushi, Ichikai-machi, Haga-gun, Tochigi (JP); Kobayashi, Tohru, Ichikai-machi, Haga-gun, Tochigi (JP); Ito, Susumu, Ichikai-machi, Haga-gun, Tochigi (JP)
(74) Representative: Kindler, Matthias, Dr. Dipl.-Chem.

(56) References cited:
- PATENT ABSTRACTS OF JAPAN vol. 014, no. 480 (C-0771), 19 October 1990 & JP 02 200191 A (OJI PAPER CO LTD), 8 August 1990
- PATENT ABSTRACTS OF JAPAN vol. 012, no. 405 (C-539), 26 October 1988 & JP 63 146790 A (SHIZUOKA PREF GOV;OTHERS: 02), 18 June 1988
- PATENT ABSTRACTS OF JAPAN vol. 005, no. 132 (C-068), 22 August 1981 & JP 56 068393 A (AGENCY OF IND SCIENCE & TECHNOL), 9 June 1981

## Description

### TECHNICAL FIELD

The present invention relates to a pectic acid lyase which is useful as a detergent, food processing agent, fiber processing agent, or the like, and to the gene thereof.

### BACKGROUND ART

Pectic acid lyase (EC 4.2.2.2) was first discovered in 1962, in a culture broth of *Bacillus polymyxa* and *Ervinia cartovora*, and requires calcium ions for reaction (Starr & Moran, Science, 135, 920-921, 1962). Generally, pectic acid lyases derived from bacteria such as those belonging to the genus *Pseudomonas*, the genus *Erwinia*, or the genus *Bacillus* have an optimum reaction pH of about 8 to 9.5 (Rombouts & Pilnik, Eco. Microbiol., 5, 227-282, 1980). Some pectic acid lyases derived from microorganisms are known to have an optimum pH of 10 to 10.5. For example, two species of enzymes produced by *Fusarium oxysporum* f. sp. *ciceri* (both have a molecular mass of 37 kDa as determined by the gel filtration method) (Arte's & Tena, Physiol. Mol. Plant Pathol., 37, 107-124, 1990) and the enzyme of *Bacillus* sp. YA-14 [molecular mass: 43 kDa as determined by the gel filtration method and sodium dodecyl sulfate-polyacrylamide electrophoresis (SDS-PAGE)] (Han *et al.*, Kor. J. Appl. Microbiol. Biotechnol., 20, 655-662, 1992) have an optimum pH of 10 to 10.5. However, these enzymes also have another peak of optimum pH at about 7 to 8. An enzyme of *Fusarium solani f. sp. pisi* (molecular weight: 29 kDa; PelB as determined by SDS-PAGE is known to have an optimum pH of about 10 (Guo *et al., J. Bacteriol*., 177, 7070-7077, 1995) and an enzyme (molecular mass: 31 kDa as determined by SDS - PAGE and 30 kDa as determined by mass spectrometry) produced by a certain strain of *Amycolata*, which belongs to Actionomycetes, is known to have an optimum pH of 10.25 (Bruhlmann, Appl. Environ. Microbiol., 61, 3580-3585, 1995).

There have been reported a relatively small number of pectic acid lyases derived from bacteria that belong to the genus *Bacillus*, which have been well studied regarding their genes and biochemistry. Since Starr and Moran discovered a pectic acid lyase having an optimum pH of 8.9 to 9.4 in culture solution of *Bacillus polymyxa*, there have been discovered an enzyme (molecular mass: about 20 kDa as determined by the gel filtration method) having an optimum pH of 8.0 to 8.5 produced by *Bacillus pumilus* (Dave & Faughn, J. Bacteriol., 108, 166-174, 1971), an enzyme (molecular mass: 31 to 35 kDa as determined by the gel filtration method) having an optimum pH of 8.5 produced by *Bacillus subtilis* (Chesson & Coidner, J. Appl. Bacteriol., 44, 347-364, 1978), and an enzyme (molecular mass: unknown) having an optimum pH of 8.5 to 9.3 produced by a certain species of thermophilic *Bacillus* (Karbassi & Luh, J. Food Scie., 44, 1156-1161, 1979). The enzyme produced by the strain *Bacillus subtilis* SO 113 has a molecular mass of 42 kDa as determined by SDS-PAGE and an optimum pH of 8.4. However, it also has another active peak at a pH of 10 or above (Nasser *et al*., Bio Chimie, 72, 689-695, 1990). Recently, Sakamoto *et al*. purified a pectic acid lyase having protopectinase activity (protopectinase-N) from Bacillus subtilis IFO 3134 (Biosci. Biotech. Biochem., 58, 353-358, 1984). The pectic acid lyase has an optimum pH of 8.0 and a molecular mass of 43 kDa as determined by SDS-PAGE and 32 kDa as determined by gel filtration.

In JP A 2200191, a DNA coding for a region involved in the expression and secretion of pectic acid lyase III (PAL III) belonging to the genus *Erwinia* is disclosed. The PALIII protein displays an optimum reaction pH of about 10.0 and a molecular weight determined by using SDS PAGE of about 36 kD.

JP A 63146790 describes a method of producing PAL, the method comprises expressing a gene derived from *Envinia carodovora subsp carodovora* EC1, by use of E. coli and obtaining the same by recombination. No sequence, neither nucleid , amino acid sequence, nor any enzymatic properties of the enzyme is described therein.

JP A 5668393 concerns a method of producing PAL by cultivating an alcalophilic *Bacillus Sp*. The pH optimum of the enzyme disclosed therein is in the range of 8.7 to 9.6.

However, these conventional pectic acid lyases exhibit low productivity, are expensive, and in addition, have drawbacks such as limited industrial application and insufficient enzymatic activities. Thus, they have not yet come to be widely used in the industry.

Accordingly, an object of the present invention is to provide a mass-producible pectic acid lyase which exhibits excellent action of degrading specifically pectin present in the cell wall of pectin-containing plants or a substance containing fragments of the cell wall, as well as to provide the gene of the pectic acid lyase.

### DISCLOSURE OF THE INVENTION

Accordingly, the present inventors have searched for microorganisms that produce pectic acid lyase in the nature and conducted studies of the gene for the enzyme, and techniques for mass production of the lyase by use of genetic engineering. As a result, they discovered a novel pectic acid lyase produced by a microorganism belonging to the genus Bacillus isolated from soil. Furthermore, they cloned its gene and established a production technology. In addition, they succeeded in gene recombination techniques to prepare mutants of the enzyme, and elucidated their characteristics, thus leading to completion of the invention.

Accordingly, the present invention provides a pectic acid lyase having an amino acid sequence of Sequence No. 1 or an amino acid sequence of Sequence No. 1 with one or more amino acids being deleted, replaced, or added, as defined in claim 1.

The present invention also provides a gene which codes for the above-mentioned pectic acid lyase, a recombinant vector containing the gene, and a transformant containing the vector.

The present invention also provides a method for the manufacture of the aforementioned pectic acid lyase, by culturing the aforementioned transformant, and collecting pectic acid lyase from the culture.

The present invention also provides a microorganism identified as *Bacillus* sp. KSM-P15 (FERM BP-6241).

### BRIEF DESCRIPTION OF THE DRAWINGS

Fig. 1 shows the DNA sequences of primer 1 and primer 2 for cloning the gene of the pectic acid lyase of the present invention.

Fig. 2 shows the DNA sequence and a deduced amino acid sequence between primer 1 and primer 2, as well as the locations of primer 3 and primer 4.

Fig. 3 shows primer 5 and primer 6 which are used for amplifying the entire gene for the pectic acid lyase of the present invention. These are primers for PCR amplification described in Example 5, and the resultant amplified fragments (about 1 kbp) are digested with *Sal* I, and subsequently ligated to pHSP64 digested with Sal I and *Sma* I.

Fig. 4 shows ligation of the gene of pectic acid lyase (BPAL) to a secretion vector (pHSP64), as well as the created secretion vector for the expression of the pectic acid lyase pHSP-A156.
- Amp :: Ampicillin-resistant marker gene
- Tet :: Tetracycline-resistant marker gene

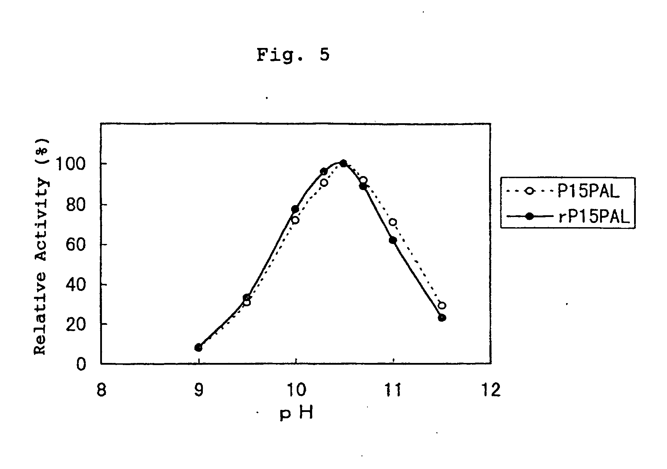

Fig. 5 shows the pH-activity curves of completely purified samples of BPAL (Example 3) and rPAL (Example 8) of the present invention. The activity is expressed in relation to the maximum activity (100%) at a pH of about 10.5 in a glycine-sodium hydroxide buffer (50 mM).
―°― : rPAL, ...•... : BPAL

Fig. 6 is an SDS-PAGE photograph of completely purified samples of the pectic acid lyase of the present invention and mutants thereof.

The arrows on the right-hand side of the protein bands indicate the molecular mass of the standard proteins for determining molecular mass. Enzymes of respective lanes are as the follows. 1 : BPAL from Example 3, 2 : rPAL from Example 8, 3 : Cys67Ala, 4 : Cys71Ala, 5 : Trp78Ala, 6 : Arg120Lys, 7 : Lys41Ala, 8 : Lys89Ala, 9 : Lys107Ala, 10 : Lys129Ala, 11 : Arg132Ala.

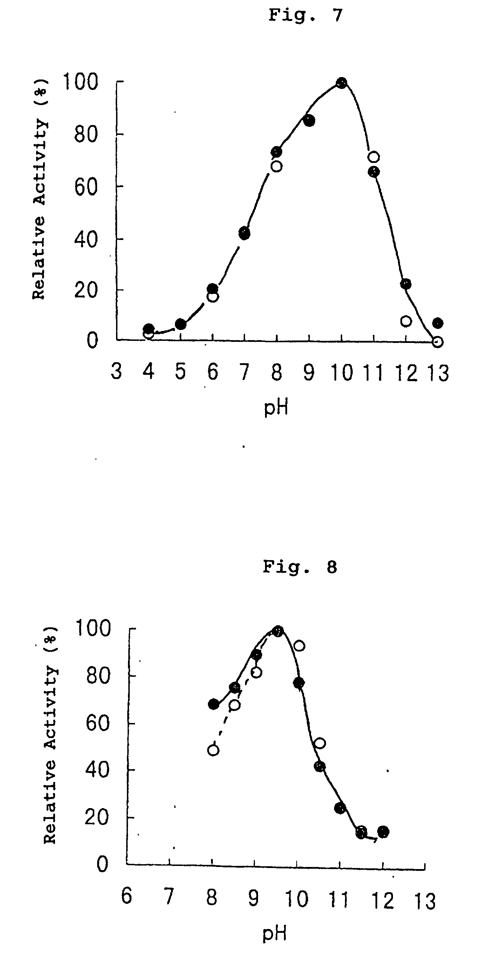

Fig. 7 and 8, show the pH dependency of pectin-release activity when pectin is released from cotton in the presence of the pectic acid lyases of the present invention (BPAL (Example 3) and rPAL (Example 8)) (in Britton-Robinson buffer). Respective enzymes were reacted with pieces of cotton in Britton-Robinson buffer (40 mM) having different pH values. The maximum protopectinase activities of BPAL and rPAL were obtained at a pH of about 10 (optimum pH), and activities are expressed in relation to the maximum activity (100%) of rPAL.
―°― : BPAL, ...•... : rPAL

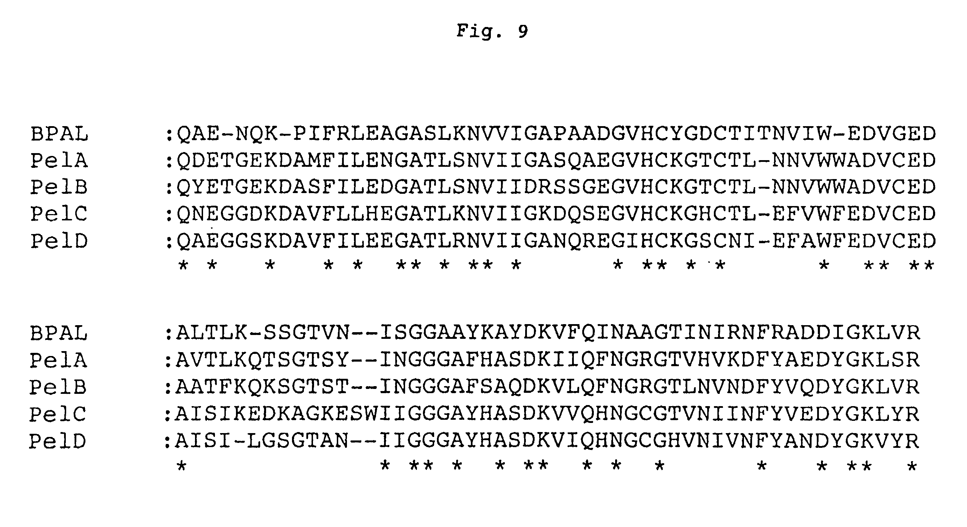

Fig. 9 shows the homology in amino acid sequence between the pectic acid lyase of the present invention and other enzymes.

The 36th to 132nd amino acids of Sequence No. 1 were used as a reference for determining homology. Amino acid residues marked with "*" represent those conserved in the five different enzymes.
- BPAL :: pectic acid lyase of the present invention
- PelA :: pectic acid lyase A from *Fusarium* *solani* f. sp. *pisi*
- PelB :: pectic acid lyase B from *Fusarium solani* f. sp. *pisi*
- PelC :: pectic acid lyase C from *Fusarium solani* f. sp. *pisi*
- PelD :: pectic acid lyase D from *Fusarium solani* f. sp. *pisi*

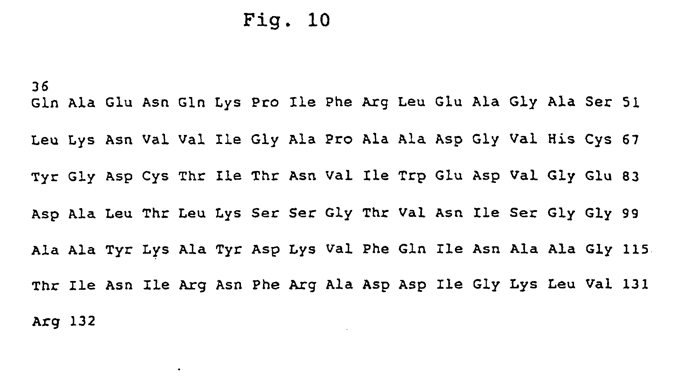

Fig. 10 shows the partial amino acid sequence used for determining the homology between the pectic acid lyase of the present invention and other enzymes.

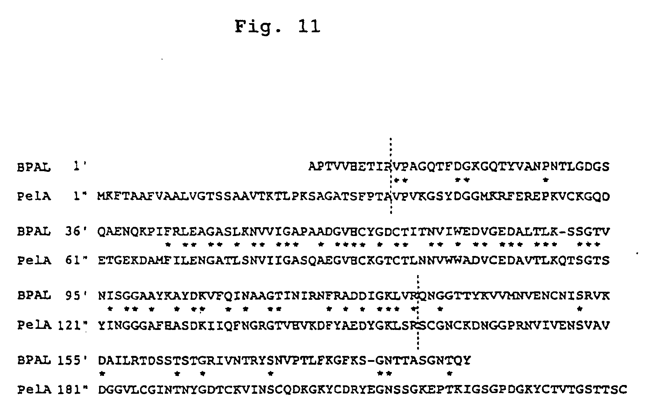

Fig. 11 shows the homology in amino acid sequence between the pectic acid lyase (BPAL) of the present invention and PelA, as determined by use of maximum-matching.

The amino acid No. 1 of PAL is counted from the N-terminal amino acid Ala residue of the mature enzyme and the amino acid No. 1 of PelA is counted from the N-terminal Met residue of the open-reading frame. The mark "*" represents the position of identity, and vertical broken lines define the homologous region as calculated by a computer.

### BEST MODE FOR CARRYING OUT THE INVENTION

The pectic acid lyase of the present invention has an amino acid sequence of Sequence No. 1, or an amino acid sequence of Sequence No. 1 with one or more amino acids being deleted, replaced, or added as defined is claim 1. No particular limitation is imposed on the deletion, replacement, or addition (hereinafter may be referred to as mutation) so long as the pectic acid lyase is not deactivated, and the mutation may preferably conserves lysine at the 107th position, lysine at the 129th position, and arginine at the 132nd position in Sequence No. 1. Also, the degree of mutation is limited as described below and the above-described 107th position, 129th position, and 132nd position are conserved. The homology is 80% or higher. One or a few amino acids may attach to the N-terminal in the amino acid sequence of Sequence No, 1. Examples of the amino acids which may attach to the N-terminal include Arg, Ala, Gly-Arg, Ala-Glu-Ala, Leu-Ala-Glu-Ala, and Gln-Ala.

The pectic acid lyase of the present invention may be manufactured by culturing a microorganism which belongs to the genus *Bacillus* (e.g., *Bacillus* sp. KSM-P15 or a mutant thereof) and which is isolated from soil, and then collecting the pectic acid lyase from the culture broth of a microorganism. Alternatively, the manufacturing method may comprise the steps of collecting the gene coding for the pectic acid lyase from the microorganism which produces the pectic acid lyase to thereby produce a recombinant vector; transforming a host cell by use of the recombinant vector; culturing the transformant; and collecting the pectic acid lyase from the culture.

The gene of the pectic acid lyase of the present invention may be cloned from a strain, e.g., KSM-P15. The cloning may be conducted by use of a conventional method, e.g., (1) the shotgun method comprising the steps of complete or partial degradation of genomic DNA by a suitable restriction endonuclease and ligation to a suitable vector, and introducing the recombinant plasmid vector into *Escherichia coli* or *Bacillus subtilis* for expression, or (2) synthesizing a suitable primer and cloning the target gene by PCR.

An exemplary nucleotide sequence of the pectic acid lyase gene of the present invention is represented by Sequence No. 1. The nucleotide sequence is not limited to Sequence No. 1, and sequences which code for the amino acid sequence of Sequence No. 1 or any of the above-described mutants may be acceptable. Preferable nucleotide sequences include the sequence identified as Sequence No. 1 or a nucleotide sequence of Sequence No. 1 with one or more bases being deleted, replaced, or added as defined in claim 1. The degree of deletion, replacement, or addition is preferably limited within the above-described mutation of amino acid sequence.

In order to create a recombinant vector containing the above-described pectic acid lyase gene, the gene is incorporated into an arbitrary vector suitable for the expression of the gene in a host of interest. Examples of the vector include pUC18, pBR322, and pUC19 for cases in which *Escherichia coli* is used as the host, and pUB110 for cases in which *Bacillus subtilis* is used as the host.

Transformation of a host by use of the thus-obtained recombinant vector is performed through a general method such as the protoplast method or the competent cell method. No particular limitation is imposed on the host, and a microorganism is preferred. Examples of the microorganism include a Gram-positive bacterium such as *Bacillus*, a Gram-negative bacterium such as *Escherichia coli*, and a fungus such as *Saccharomyces* or *Aspergillus*.

The pectic acid lyase of the present invention may be obtained through culturing the obtained transformant, e.g., the steps of culturing by use of the above-described KSM-P15 strain, collecting, and purifying. When the pectic acid lyase of the present invention is produced by use of the strain KSM-P15 (or the above-described transformant), the strain may be inoculated to a culture medium containing an assimilatory carbon source, nitrogen source, and other essential nutrients and then cultured through a customary method.

The pectic acid lyase may be collected from the thus-obtained culture and purified through generally employed methods for collection and purification of enzymes.

The enzyme-containing solution may be used with or without further purification, crystallization, or granulation through known methods.

The thus-obtained pectic acid lyase has an amino acid sequence as described above and strongly reacts with protopectin. The optimum reaction pH is 10.3-10.7, although the pectic acid lyase is not particulalrly limited thereto, so long as it exhibits pectic acid lyase activity.

The following is a more detailed description of enzymological characteristics of the pectic acid lyase of the present invention.
- (1) Action: To cleave α-1,4-bond scission of pectic acid (polygalacturonic acid) via β-elimination in an *endo* manner and to provide a double bond at the C4-C5 position of the non-reduced end to form unsaturated digalacturonide or unsaturated oligo-galacturonide.
- (2) Substrate specificity: To act on protopectin, pectic acid (polygalacturonic acid), acid-soluble pectic acid, and pectin.
- (3) Optimum pH: pH 10.3-10.7 (50 mM glycine-NaOH buffer)
- (4) Optimum temperature: 45-55°C (pH 10.5, 50 mM glycine-NaOH buffer)
- (5) Molecular mass: approximately 20.3 ± 1 kDa (as measured by sedimentation equilibrium; approximately 26 kDa as measured by SDS PAGE)
- (6) Isoelectric point: pH 9.5-10.5
(isoelectric focusing PAGE)

Each of the above enzymalogical values contains a value for variant strains. As far as KSM-P15 strains are concerned, the optimum temperature is 50∼55° C, molecular mass is about 20∼21 kda, and isoelectric point is around pH10.3.

### EXAMPLES

The present invention will next be described in detail by way of examples, which should not be construed as limiting the invention thereto.

### Example 1 : Screening for a bacterium producing pectic acid lyase

Soils from various places in Japan and suspended in sterilized water or strains stocked in the microorganism-collection of Kao Corporation were applied on an agar plate culture medium containing polygalacturonic acid and cultured at 30°C for 3-5 days. When bacteria had been grown, 1% (w/v) CATB (cetyltrimethylammonium bromide) solution was poured into the culture medium, and the resultant medium was allowed to stand for ten minutes. Bacteria which formed clear zones around colonies due to decomposition of polygalacturonic acid were selected and subjected to a test for productivity of pectic acid lyase. In this manner, *Bacillus* sp. KSM-P15 strain was selected as a pectic acid-lyase-producing bacterium.

Mycological properties of the strain KSM-P15 will be described in the following.

### A Morphological properties

| | | |
|---|---|---|
| (a) | Shape and size of cell | rod (0.3-0.5) × (1.6-2.1) µm |
| (b) | Polymorphism | no |
| (c) | Motile | yes |
| (d) | Spore (size, shape, location, | swollen) (0.6-0.7) × (1.2-1.4) µm, central to subterminal, swollen |
| (e) | Gram staining | positive |
| (f) | Acid-fastness | negative |
| (g) | Growth on broth-agar plate medium | yellowish white, punetiform, raised, entire colony formation |
| (h) | Litmus milk | slightly red without coagulation |

### B Physiological properties

| | | |
|---|---|---|
| (a) | Reduction of nitrate | positive |
| (b) | Denitrification | negative |
| (c) | MR test | positive (pH 5.5) |
| (d) | VP test | positive |
| (e) | Indole formation | negative |
| (f) | Hydrogen sulfide formation | negative |
| (g) | Starch hydrolysis | positive |
| (h) | Utilization of citric acid | positive |
| (i) | Utilization of inorganic nitrogen | no Utilization of nitrate salt or ammonium salt |
| (j) | Pigment formation | no |
| (k) | Urease | negative |
| (l) | Oxidase | positive |
| (m) | Growth temperature range | 20°C-45°C |
| (n) | Growth pH range | pH 7-10 |
| (o) | Oxygen effect on growth | growth under anaerobic conditions |
| (p) | OF test | growth, no color change |
| (q) | Gas formation from glucose | no |
| (r) | Sodium chloride-fastness | no growth on medium containing 3% sodium chloride |
| (s) | Acid formation from sugar | acid generation from galactose, xylose, arabinose, sucrose, glucose, mannitol, mannose, trehalose, lactose, glycerin, maltose, fructose, raffinose, and melibiose and no acid formation from inositol or sorbitol |

A study of the above-described mycological properties based on the description in "Berger's Manual of Systematic Bacteriology" (Williams & Wilkins Co., 1984) reasonably suggested that this strain belongs to *Bacillus circulans*, which is a strain having many variants. However, the present strain has properties which did not completely accord with those of *Bacillus circulans*, and therefore is a novel microorganism. Consequently, this strain, a new microorganism, was deposited with the National Institute of Bioscience and Human Technology, Agency of Industrial Science and Technology as *Bacillus* sp. KSM-P15 (FERM BP-6241).

### Example 2 : Cultivation of strain KSM-P15

Strain *Bacillus* sp. KSM-P15 obtained through the above-described screening process was subjected to shaking culture at 30°C for two days in a Sakaguchi flask containing a liquid culture medium (50 ml). The composition of the culture medium was 3% (w/v) of polypeptone S, 0.5% of a yeast extract, 1% of a fish meal extract, 0.15% of dipotassium hydrogenphosphate, 0.001% of calcium chloride, 0.5% of pectin, and 0.5% of sodium carbonate (separately sterilized).

### Example 3 : Purification of pectic acid lyase derived from strain KSM-P15

A culture broth of *Bacillus* sp. KSM-P15 was centrifuged (12,000 × g, 15 minutes) and ammonium sulfate was gradually added to the resultant supernatant (1,250 ml) at 90% saturation with gentle stirring. The solution was allowed to stand for 24 hours at 5°C and centrifuged (12,000 × g, 15 minutes) for collection of the precipitates. The precipitates were dissolved in a small amount of 10 mM Tris-HCl buffer containing 1 mM calcium chloride (pH 7.0) and were subjected to dialysis against the same buffer for 24 hours. Every 100 ml portion from the retentate (900 ml) was applied to a Super Q Toyopearl 650M (product of Tosoh Corp.) column (2.5 x 10 cm) pre-equilibrated with 50 mM Tris-HCl buffer containing 1 mM calcium chloride solution (pH 7.5). The proteins eluted with the equilibration buffer, exhibited activity, and were collected (1,100 ml). Subsequently, every 200 ml portion of the above-described non-adsorbed fractions was injected to a Bio Cad 60 HPLC system (product of Nihon Perceptive Co.) equipped with a sulfopropyl column pre-equilibrated with 20 mM calcium chloride (pH 7.0). Protein was eluted by a linear gradient of 0 M-0.2 M sodium chloride in the equilibration buffer. Pectic acid lyase activity was detected as a single peak at approximaterly 0.1 M of sodium chloride and protein was homogeneous as judged by SDS-PAGE. The above-described operations were repeated to thereby obtain a purified pectic acid lyase (hereinafter the pectic acid lyase obtained from this step may be referred to as BPAL). The activity yield was about 60% and 50 folds of purification.

### Example 4 : Enzymatic properties of pectic acid lyase from strain KSM-P15 and method for determination

### (1) Standard enzymatic activity

0.5 M glycine-NaOH buffer (pH 10.5) (0.3 ml), 6 mM calcium chloride (0.3 ml), and deionized water (1.7 ml) were placed in a test tube, which was then incubated at 30°C for 5 minutes. An appropriately diluted enzyme solution [0.1 ml, diluted with 50 mM Tris-HCl buffer containing 1 mM calcium chloride (pH 7.5)] was added to the test tube, which was further incubated at constant temperature for 5 minutes. An aqueous solution of polygalacturonic acid (0.6 ml, 1% (w/v), product of ICN Biomedicals Co., Lot 14482, pH 6.8 adjusted with sodium hydroxide solution) was added to the enzyme solution to initiate reaction, and the mixture was incubated at 30°C for 10 minutes. The reaction was terminated by the addition of 3 ml of 50 mM HCl. The quantity of unsaturated Oligogalacturonic acid formed in the reaction was determined through measuring the absorbance at 235 nm and calculating with the molar extinction coefficient of unsaturated digalacturonide (4600 M⁻¹ cm⁻¹, Hasegawa & Nagel, *J. Food Sci*., 31, 838-845, 1966). A test blank was prepared as follows: 50 mM hydrochloric acid (3 ml) was added to a reaction mixture treated without being incorporated with the enzyme solution, and subsequently an enzyme solution (0.1 ml) was added thereto. One unit of enzyme (1U) is defined as the amount of enzyme that produces unsaturated Oligogalacturonide equivalent to 1 µmol of unsaturated digalacturonide per minute under the above-described reaction conditions.

### (2) Optimum pH

The optimum pH for the reaction was investigated through use of 50 mM Tris-HCl buffer (pH 7-9) or 50 mM glycine-NaOH buffer (pH 8.5-11.0). The enzyme exhibits the highest rate of reaction in glycine-NaOH buffer (pH 10.5). At pH 10.3-10.7, the activity is 90% or more of the maximum activity, and the activity is 70% or more of the maximum activity between pH 10 and 11.

### (3) Optimum temperature

The optimum temperature for the reaction was investigated through use of 50 mM glycine-NaOH buffer (pH 10.5) at temperatures between 10°C and 70°C inclusive. The results show that the enzyme acts in a wide temperature range of 10°C-65°C, with 50-55°C being optimum.

### (4) Molecular mass

a. The molecular mass of the enzyme obtained through sedimentation equilibrium experiment is 20.3 ± 1.0 kDa.
b. The molecular mass of the enzyme was estimated to be approximately 26 kDa by SDS-PAGE (15% gel). A molecular weight marker (SDS-PAGE Molecular Weight Standards, Low Range, product of Bio-Rad) was used as a standard protein.

### (5) Isoelectric point

The isoelectric point of the enzyme was determined to be approximately pH 10.3 by the isoelectric focusing PAGE by use of 5% polyacrylamide gel containing an ampholyte of the pH 8-10.5 (Pharmalyte, product of Pharmacia).

### (6) Amino terminal sequence

The enzyme was blotted on a ProSorb filter (Perkin-Elmer Co.) and was analyzed by use of a protein sequencer (model 674, Applied Biosystem Co.), to determine the amino acid sequence up to the 20th amino acid; i.e., APTVVHETIRVPAGQTFDGK.

### (7) Action against protopectin

Finely-cut cotton fiber pieces (1-2 mm by 1-2 mm) obtained from working gloves for quality control (40 interlock bleached cotton fabric, product of Nakada Hisakichi Store) was used as a substrate (10 mg, 1%, w/v). The substrate was suspended in 100 mM glycine-NaOH buffer containing 0.6 mM calcium chloride solution (pH 10.5), and the enzyme was added thereto. The mixture was reacted at 30°C and 120 rpm for 60 minutes. The resultant solution was centrifuged and the quantity of pectic acid isolated in the supernatant was determined by use of carbazole-sulfuric acid method (Sakai, *Methods Enzymol*., 161, 335-350, 1988). 20-50 µg (as galacturonic acid) pectic acid was released from 10 mg of cotton fibers when the enzyme was used in an amount of 0.1 U. This indicates that the enzyme reacted with protopectin on the surface of cotton fiber. Furthermore, the enzyme has proven to have protopectinase activity assigned to A type, judging from the increase in absorbance of the supernatant at 235 nm. When acid-soluble pectic acid prepared according to the Ward and Fogarty (*J. Gen. Microbiol*., 73, 439-446, 1972) was used as a substrate, the substrate degraded at a rate of reaction nearly equal to the degradation rate of pectic acid.

### (8) Degradation mechanism of substrate

Activity of decreasing viscosity was measured in glycine-NaOH buffer (pH 10.5) according to Crawford and Kolattukdy (*Arch. Biochem*. *Biophys*., *258*, 196-205, 1987). The viscosity decreases with reaction time, i.e., initial viscosity (taken as 100%) dropped gradually to 60% after 30 minutes and to 40% after 60 minutes. In addition, glycosidic linkage cleaved was only 2% of the total linkage after 60 minutes; consequently the enzyme has proven to be an endo-type pectic lyase.

### (9) Effects of chemical reagents

Various chemical reagents were added to 50 mM Tris-HCl buffer (pH 7.5). An enzyme solution was added thereto. After the mixture was incubated at 30°C for 1 hour, the residual activity was measured through the standard conditions of assay. The results show that the enzyme is not inhibited by surfactants (0.01-0.1%), chelating agents (0.15-0.20%), or other compounds.

### Example 5 : Internal amino acid sequence of a pectic lyase derived from strain KSM-P15

The enzyme was treated with lysyl endopeptidase. The internal peptides were fractionated from the treated solution by use of an automated C-terminal fragment fractionator (CTFF-1 : Shimadzu Corp.). The sample was blotted on a ProSorb filter and the N-terminal amino acid sequence was determined by use of a protein sequencer to thereby obtain the sequence of VVIGAPAADGVH.

### Example 6 : Cloning of the gene for pectic acid lyase

### (1) Preparation of genomic DNA of strain KSM-P15

Strain KSM-P15 was cultured aerobically in a liquid culture medium with shaking and the culture broth was centrifuged to collect cells. The genomic DNA was prepared from the obtained cells according to Saito and Miura (*Biochim*. *Biophys. Acta*, 72, 619-629, 1963).

### (2) Preparation of primers

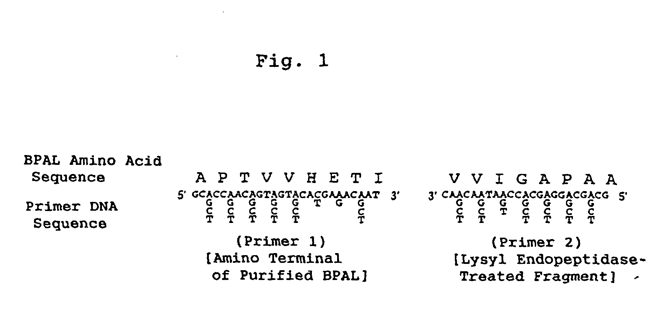

Primer 1 and primer 2 were synthesized based on the results of Example 4 (6) and Example 5 (Fig. 1).

### (3) Cloning

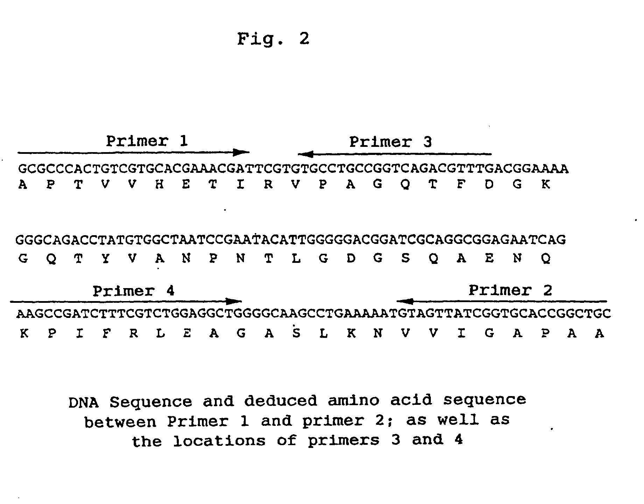

PCR was conducted by use of primer 1 and primer 2, and genomic DNA (0.5 µg) of *Bacillus* sp. KSM-P15 as a template. The obtained amplified fragment was purified with a PCR fragment purification kit (Boehringer Mannheim) and cloned by introducing the fragment to the *Sma* I site of a plasmid vector pUC19. A partial gene sequence of the target pectic acid lyase was detected in the determined nucleotide sequence of several obtained clones and the amino acid sequence was also deduced (See Fig. 2).

Subsequently, inverse PCR was conducted in order to amplify an upstream region and a downstream region of the above-described PCR-amplified fragment. The nucleotide sequences observed in Fig. 2, primer 3, and primer 4 were used. A genomic DNA (1 µg) of strain KSM-15 was predigested with Pst I, extracted with phenol/chloroform, and treated with T4 DNA ligase to form an intramolecular bond (circularized DNA bond) so as to provide a template. PCR was conducted by use of a Long Template System PCR kit (TaKaRa Co.). An amplified fragment of about 2.0 kbp was detected and the the DNA fragment was sequenced directly. Thus, the amino acid sequence and nucleotide sequence (Sequence No. 1) of pectic acid lyase comprising 197 amino acids from the N-terminal amino acid sequence to the amino acid before the termination codon (TAA). From the sequence, the molecular weight of the pectic acid lyase (secretion-type matured enzyme) produced from strain KSM-P15 is deduced to be 20924 Da (about 21 kDa).

### Example 7

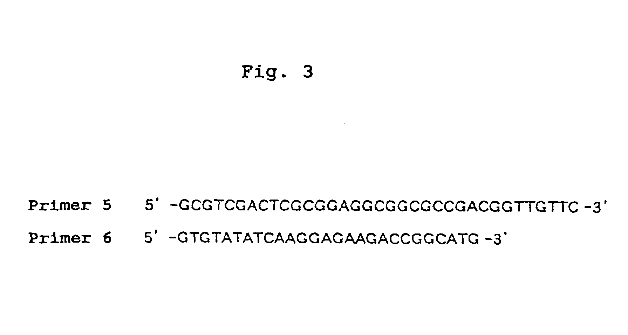

Primer 5 (Fig. 3) was designed so as to link the deduced signal peptidase-recognizing sequence of Ala-Glu-Ala located just upstream of N-terminal amino acid Ala of the pectic acid lyase produced by strain KSM-P15 and the signal sequence derived from the host vector employed. Primer 6 (Fig. 3) was designed to have a sequence of 26 bp located in a downstream region (by 372 bp) from the termination codon (TAA) of the gene for the pectic acid lyase.

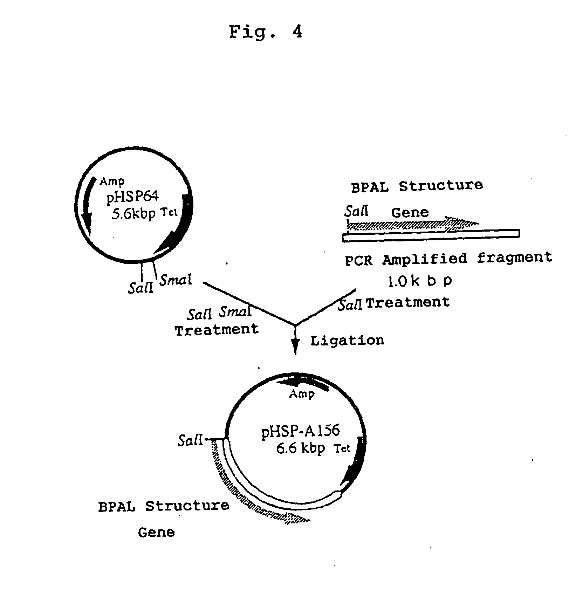

PCR was conducted by use of primer 5 and primer 6, and genomic DNA of strain KSM-P15 as a template, to thereby amplify the DNA fragment to about 1 kbp. The DNA fragment was digested with Sal I, and ligated to pHSP64 (Sumitomo et *al., Biosci, Biotech. Biochem*., 56, 872-877, 1992) which had been cut with *Sal* I and *Sma* I by use of a ligase (see Fig. 4).

The resultant recombinant plasmid was transformed to *E. coli* HB101 Cells and the transformants grew on the agar plate medium of Example 1 to form clear zones around colonies. The obtained recombinant plasmid of the present invention that codes for the gene of the present invention was named pHSP-A156.

Subsequently, pHSP-A156 was introduced into *Bacillus* *subtilis* ISW1214 cells and the transformants were cultured in a liquid medium at 30°C for three days, to thereby extracellulary produce a pectic acid lyase in a considerably large amount.

### Example 8

A crude enzyme solution of the pectic acid lyase obtained in Example 7 was completely purified in accordance with Example 3 (the obtained pectic acid lyase may be hereinafter referred to as rPAL). The N-terminal amino acid sequence of the enzyme was determined in a manner similar to that described in Example 4. A sequence containing APTVVHETIRVPAGQTFDGK was detected.

### Example 9

The procedure described in Example 4 was repeated by use of rPAL obtained in Example 8 to investigate the optimum pH for the reaction, molecular mass, and degradation of protopectin. rPAL was also found to have properties similar to those of the pectic acid lyase from Example 3.

### Example 10

The pH dependency of the activity to release pectin from cotton fibers (interlock cotton fabric gloves) due to the pectic acid lyases obtained in Example 3 and Example 8 was verified. The quantity of released pectin was determined at different pHs by the carbazole-sulfuric acid method. As shown in Fig. 7 and 8, both enzymes exhibited an optimum pH for the protopectinase activity around at pH 10 in 40 mM Britton-Robinson buffer, and the two relative activity curves matched almost completely. In both cases, the maximum activity in 0.1 M glycine-NaOH buffer under similar conditions was observed around at pH 9.5. These results indicate that the pectic acid lyase, which is the gene product of the present invention, and wild-type pectic acid lyase from *Bacillus* sp. ksM-P15 exhibit alkaline protopectinase activity as well as alkaline pectic acid lyase activity.

### Example 11

Effects of a specific inhibitor were investigated by use of the pectic acid lyases from Example 3 and Example 8. Briefly, an enzyme sample was treated with a specific inhibitor (appropriate concentration) in 50 mM Tris-HCl buffer (pH 7.5) at 30°C for one hour and the residual activity was measured. The percentage inhibitions of the two enzymes were about 75-80% in the case of 2,4,6-trinitrobenzenesulfonic acid (1 mM), and 30-40% in the case of N-bromosuccinimide (0.1 mM). p-Chloromercuribenzoic acid (0.5 mM), N-ethylmaleimide (1 mM), iodoacetic acid (1 mM), diethyl pyrocarbonate (1 mM), 5,5'-dithiobis(2-nitrobenzoic acid) (1 mM), dithiothreitol (1 mM), and glyoxal (1 mM) slightly inhibited or did not completely inhibit the enzymes.

### Example 12

The effect of H₂O₂ on the activity of pectic acid lyases of Example 3 and Example 8 was investigated. Each enzyme solution (250 µl), H₂O₂ (0-30%, 50 µl), and a 50 mM Tris-HCl buffer (pH 7.5, 250 µl) were mixed, and the mixture was incubated at 30°C for 30 minutes. Then catalase (product of Boehringer Mannheim, 0.5 mg/ml, 100 µl) was added to the solution, and the mixture was maintained for 5 minutes to decompose H₂O₂. When the residual activity of the treated solutions was measured by a standard conditions of assay, both enzymes were found to not be inactivated even in the presence of 3% H₂O₂.

### Example 13

Homology in the amino acid sequence of the pectic acid lyase of the present invention and known pectic acid lyases was investigated.

The homology to other enzymes was investigated with respect to amino acid numbers between 36 and 132 in Sequence No. 1. As shown in Fig. 9, PelA (Crawford & Kolattukdy, *Arch. Biochem. Biophys*., 258, 196-205, 1987, Gonzalez-Candelas & Kolattukdy, *J. Bacteriol*., 174, 6343-6349, 1992), PelB (Guo *et al., J. Bacteriol*., 177, 7070-7077, 1995), PelC (Guo *et al., Arch. Biochem*. *Biophys*., 323, 352-360, 1995), and PelD (Guo *et al*., *Arch. Biochem. Biophys*., 332, 305-312, 1996) from fungi *Fusarium* exhibited homologies of merely 54.6%, 52.6%, 50.5%, and 54.6%, respectively. Consequently, enzymes exhibiting homology higher than the above values, when appropriately aligned to the amino acid sequence of the present invention in amino acid numbers between 36 and 132, are to be included in the present invention. In other words, the homology of amino acid sequence being included in the present invention to the amino acid sequence (amino acid numbers between 36 and 132 in Sequence No. 1) described in Fig. 10 is preferably 55.7% or more. The homology in amino acid sequence between PelA having the highest homology among all and the pectic acid lyase of the present invention decreases when matching is conducted by use of a conventional homology matching program. In other words, PelA exhibits only 45.5% homology to the amino acid sequence of the enzyme of the present invention from amino acid numbers 11 (Val) to 132 (Arg) and 33.0% to the entire amino acid sequence (amino acid number 1 (Ala) to 197 (Tyr)) (Fig. 11).

### Example 14

The crystal structures of the pectic acid lyases derived from *Erwinia chrysanthemi* and *Batillus subtilis* have been conventionally analyzed by Yoder *et al*. (*Science*, 260, 1503-1507, 1993), Lietzke *et al*. (*Plant Physiol*., 106, 849-862, 1994) and Pickersgill *et al*. (*Struct. Biol*., 1, 717-723, 1994). As a result, there have been specified the Ca²⁺-binding site (acidic amino acid residue) relating to the expression of activity, the substrate-binding site (basic amino acid residue), a cysteine residue maintaining the structure, etc. However, the enzyme of the present invention has low homology to conventional pectic acid lyases when compared in the primary structure, and no homology was observed to the above-described sequence containing important amino acid residues. Therefore, the characteristics of the enzyme of the present invention may be more clearly elucidated by specifying the important amino acid residues relating to expression of activity or maintaining the molecular structure in the amino acid sequence described in Fig. 10. The details will be described in the following.

### (1) Production of a mutant of the pectic acid lyase of the present invention

An arbitrary variant protein of the gene of the present invention was artificially generated according to the protocol of Site-directed Mutagenesis System Mutan-Super Express KM kit (TaKaRa Co.).

### (a) Introduction of the gene of the present invention into plasmid vector pKF19k for mutation

A fragment (about 1.5 kbp) containing a structural gene from a high-expression promoter region on pHAPAL harboring the gene for the pectic acid lyase of the resent invention was amplified by PCR, and the amplified fragment was inserted into the SmaI site of the plasmid pKF19k (this was designated as pKFPAL).

### (b) PCR for introducing mutation

PCR was conducted by use of a template DNA (pKFPAL), a selection primer, a phosphated primer for mutation, 10×LA PCR BufferII, a dNTP mixture solution, and TaKaRa LA Taq. The amplified DNA was purified by use of a PCR fragment purification kit (Boehringer Mannheim), and subjected to transformation.

### (c) Introduction into Escherichia coli MV1184 and verification of mutation

The PCR product was introduced into *E. coli* MV1184 competent cells (TaKaRa Co.). A plasmid DNA was prepared from a few colonies grown on an LB agar plate containing kanamycin (50 µg/ml), and the mutated gene was selected through conducting sequence analysis.

The following are primer sequences used for inducing mutation. (The amino acid numbers correspond to those in Sequence No. 1, the left-side to the symbols indicates an intrinsic amino acid residue, and the right-side to the symbols indicates a variant amino acid residue.) (The underlined triplet codon in the nucleotide sequence codes for an amino acid to be substituted.)

Subsequently, *Bacillus subtilis* was transformed through the mutant to produce extracellulary in a large amount of a mutant pectic acid lyase. The mutant pectic acid lyase was then purified and the enzymatic activity was measured (see Example 7).

### (2) Verification of the activity of the mutant proteins

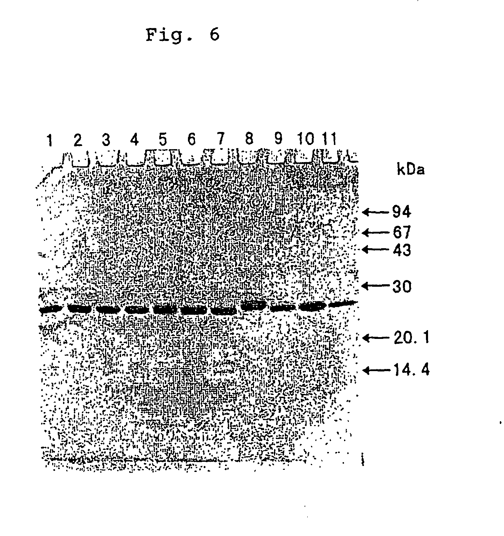

The obtained rPAL, Lys41Ala, Cys67Ala, Cys71Ala, Lys89Ala, Lys107Ala, Lys129Ala, and Arg132Ala were produced extracellulary maintaining molecular structure of each enzyme, they migrated same distance as that of BSPAL on SDS-PAGE (Fig. 6). These mutant proteins were purified (see Example 3) and their activity was verified though the standard conditions of assay described in Example 1.

**Table 1**

| Activity of variant enzymes (I) | |
|---|---|
| Mutant enzyme | Specific activity of purified enzyme (U/mg protein) |
| Cys67Ala | 183 |
| Cys71Ala | 201 |
| Enzyme of Example 8 (rPAL as control) | 220 |

As described in Table 1, even when each Cys residue in Sequence No. 1 was replaced by Ala residue, the enzymes had sufficient activity and the molecular structures were maintained, as judged by SDS-PAGE. The results indicate that these Cys residues are not essential to express the enzymatic activity and maintain molecular structure regardless of forming disulfide bond, proven by the fact that the enzymatic activity is not inhibited by SH-inhibitor and SH-compounds (Example 11). Also, H₂O₂ often strongly oxidizes a sulfur-containing amino acids, Met and Cys, to inhibit the enzymatic activity, whereas the enzyme of the present invention is not inhibited by high-concentration H₂O₂ (Example 12).

**Table 2**

| Activity of variant enzymes (II) | |
|---|---|
| Variant enzyme | Specific activity of purified enzyme (U/mg protein) |
| Lys41Ala | 212 |
| Lys89Ala | 48 |
| Lys107Ala | <1 |
| Lys129Ala | <1 |
| Enzyme of Example 8 (rPAL as control) | 220 |

The replacement of four Lys residues in Sequence No. 1 with other amino acid residues definitely affects the activity of the enzyme of the present invention. As described in Table 2, a remarkable decrease was observed in the specific activity of the mutant enzymes, including Lys89Ala, Lys107Ala, and Lys129Ala. In particular, the mutant enzymes of Lys107Ala and Lys129Ala did not exhibit the enzyme activity even though their molecular structures were maintained, as judged by SDS-PAGE. The results indicate that the Lys residues at these location are essentially responsible for the expression of the enzymatic activity. Also, Lys89Ala still had residual activity, which showed about 20% of the specific activity of the recombinant pectic acid lyase of Example 8 (rPAL). This mutant enzyme was purified to homogeneity as judged by SDS-PAGE, and the molecular mass of the mutant enzyme was same as that of the other mutant and wild-type enzymes. Consequently, the results indicate that the Lys residues effected the pectic acid lyase activity through a certain mechanism. The importance of catalysis related to the Lys residues has not been known for a pectic enzymes including pectic acid lyases which have ever reported, except the pectic acid lyace from *Erwinia chrysanthemi* EC16, which has no homology to the enzyme of the present invention (Kita *et al., J. Biol. Chem*., 271, 26529-26535, 1996). The essential role of the Lys residue has confirmed that the enzymatic activity was strongly inhibited by trinitobenzenesulfonic acid as a specific inhibitor to Lys residue (Example 11).

On the other hand, the Lys residue at position 41 in Fig. 10 is also conserved in amino acid squence of PelA to PelD (see Sequence No. 1). The Lys residue at this position might relate to the catalytic action. It migrated as a single protein band on SDS-PAGE as shown in Fig. 6, the specific activity of the enzyme was identical to that of a wild-type enzyme. Therefore, the results indicate that the Lys residue at the position has no direct relation to the catalytic action of the enzyme of the present invention.

Furthermore, the Arg residue at position 132 in the amino acid sequence is conserved in the amino acid sequence of the other enzymes (Fig. 9). This indicates that the Arg residue, which belongs to a basic amino acid same as the Lys residue, might have direct relation to the catalytic action of the enzyme of the present invention.

**Table 3**

| Activity of variant enzymes (III) | |
|---|---|
| Variant enzyme | Specific activity of purified enzyme (U/mg protein) |
| Arg132Ala | <1 |
| Pectic acid lyase of Example 7 (rPAL as control) | 237 |

In fact, surprisingly, replacement of the Arg residue at location 132 with, for example, Ala, results in complete loss of the enzymatic activity as described in Fig. 6 and Table 3. The Arg residue at this location must be considered to be an essential residue for expression of the enzymatic activity in the case of the above-described Lys residues. The effect of 2,3-butanedione as an arginine-modifier on the activities of both wild-type and reconbinant enzyme was therein investigated. The enzymes were incubated at 30° C for 30 minutes with 2,3-butanedione in 0.1 M borate buffer (pH 9.5), and the residual activities were measured. Both enzymes were inhibited by 40% or more. The results suggest that the Lys at positions 107 and 129 may be replaced with other basic amino acid residue such as Arg or His. Also, replacement of the Arg at position 132 with another basic amino acid residue might maintain the activity. Therefore, mutant enzymes in which the Lys and Arg were replaced with other basic amino acids were prepared through the above-described method by use of the following primers and were then completely purified.

The results show that replacement of at least the Lys at position 107 and location 129 with Arg and His causes complete loss of the enzymatic activity and substantially no activity is observed through replacement of the Arg at position 132 with Lys.

**Table 4**

| Activity of variant enzymes (IV) | |
|---|---|
| Variant enzyme | Specific activity of purified enzyme (U/mg protein) |
| Lys107Arg | <1 |
| Lys107His | <1 |
| Lys129Arg | <1 |
| Lys129His | <1 |
| Arg132Lys | <1 |
| Arg132His | <1 |
| Pectic acid lyase of Example 8 (rPAL control) | 237 |

Accordingly, at least the Lys at positions 107 and 129 and the Arg at position 132 may not be replaced with other amino acid residues; i.e, each of the Lys 107, Lys 129, and Arg 132 residues might specifically relate to express the enzymatic activity..

On the other hand, only one Trp residue exists in Sequence No. 1 and the Trp residue is well-conserved in the amino acid sequences of PelA to PelD (Fig.9). Actually, the enzyme of the present invention is remarkably inhibited by N-bromosuccinimide, which is a specific oxidizing agent to tryptophane. Consequently, the Trp (position 78) was replaced with an Ala, Phe, and Tyr by use of the below-described primers, respectively.

The mutant enzymes obtained were homogeneously purified as judged by SDS-PAGE (Fig. 6). The results show that the replacement with the Ala causes the complete loss of the enzymatic activity. On the other hand the enzymatic activity is maintained as same that of wild-type enzyme through replacement with the Phe or the Tyr. The activity is not observed through the replacement with other amino acid residue except for Tyr or Phe, indicating the requirement of an aromatic amino acid residue at position 78.

**Table 5**

| Activity of variant enzymes (V) | |
|---|---|
| Variant enzyme | Specific activity of purified enzyme (U/mg protein) |
| Trp78Ala | <1 |
| Trp78Phe | 213 |
| Trp78Tyr | 225 |
| Pectic acid lyase of Example 8 (rPAL as control) | 230 |

As shown in Fig. 10, Arg at position 120 in the amino acid sequence of the present invention is not identical to amino acid of PelA to PelD. It is interesting that this amino acid residue may be replaced with another basic amino acid residue or not. Therefore, completely purified mutant enzymes were prepared through the above-described method using the below primer.

The activity of the mutant enzyme is observed at equal level of that of rPPAL, indicating that at least Arg 120 can be substituted with Lys in the substitution of positions 107 and 129 and the Arg residue at position 132 (Fig. 6 and Table 6).

**Table 6**

| Activity of variant enzymes (VI) | |
|---|---|
| Variant enzyme | Specific activity of purified enzyme (U/mg protein) |
| Arg120Lys | 216 |
| Pectic acid lyase of Example 8 (rPAL as control) | 221 |

From the above results, it is concluded that the 107th Lys, 129th Lys, and the 132nd Arg within the amino acid sequence of the pectic acid lyase of the present invention are strongly responsible for the expression of enzymatic activity. Therefore, mutation is preferably devised so as to conserve these positions.

### INDUSTRIAL APPLICABILITY

The present invention provides a completely novel pectic acid lyase having an optimum reaction pH in an alkali region, a gene thereof, and a manufacturing method thereof. The enzyme also acts on a pectic substance and vegetable fibers and is preferably used as a detergent, a food-processing agent, or a fiber-processing agent.

## Claims

1. A pectic acid lyase having an amino acid sequence of SEQ NO 1 or an amino acid sequence having 80% or more homology to SEQ NO 1 between amino acids Nos. 36-132 and having the following properties:
(1) Action
to cleave α-1,4-bond scission of pectic acid (polygalacturonic acid) via β-elimination in an endo manner and to provide a double bond at the C4-C5 position of the non-reduced end to form unsaturated digalacturonide or unsaturated oligo-galacturonidea
(2) Substrate specificity
to act on protopectin, pectic acid (polygalacturonic acid), acid-soluble pectic acid, and pectin;
(3) Optimum pH
pH 10.3 - 10.7 (50 mM glycine-NaOH buffer);
(4) Optimum temperature
45 - 55°C (pH 10.5, 50 mM glkycine-NaOH buffer)
(5) Molecular mass
approximately 20.3 ± 1 kDa (as measured by sedimentation equilibrium;
approximately 26 kDa as measured by SDS PAGE);
(6) Isoelectric point
pH 9.5 - 10.5
(isoelectric focusing PAGE).

2. The pectic acid lyase according to claim 1, which is derived from the genus Bacillus.

3. The pectic acid lyase according to claim 1 or 2, which is obtainable from Bacillus sp. KSM-P15 (FERM BP-6241).

4. The pectic acid lyase according to claim 1 wherein the deletion, replacement, or addition of one or more amino acids is performed so as to conserve the 107^{th} Lys, 129^{th} Lys, and the 132^{nd} Arg within the amino acid sequence of Sequence No. 1.

5. A gene coding for the pectic acid lyase according to any one of claims 1 through 4.

6. A recombinant vector containing a gene as described in claim 5.

7. A transformant containing the recombinant vector as described in claim 6.

8. The transformant according to claim 7 which is created by use of a microorganism as a host.

9. A method for the manufacture of a pectic acid lyase as described in any of claims 1 through 3, **characterized by** culturing a transformant as described in claim 7 or 8 and collecting pectic acid lyase from the culture.

10. Bacillus sp. KSM-P15 (PERM BP-6241).

## Patentansprüche

1. Pectinsäurelyase mit einer Aminosäuresequenz der SEQ ID NO: 1 oder einer Aminosäuresequenz mit 80 % oder mehr Homologie zu SEQ ID NO: 1 zwischen den Aminosäuren Nrn. 36-132 und mit den folgenden Eigenschaften:
(1) Wirkung:
zur Spaltung einer α-1,4-Bindungsspaltung der Pectinsäure (Polygalacturonsäure) über β-Eliminierung in Endo-Weise und zur Bereitstellung einer Doppelbindung an der C₄-C₅-Stellung des nicht-reduzierten Endes, um ein nicht-gesättigtes Digalacturonid oder nicht-gesättigtes Oligogalacturonid zu bilden;
(2) Substratspezifität
zur Wirkung an Protopectin, Pectinsäure (Polygalacturonsäure), säurelöslicher Pectinsäure und Pectin;
(3) optimaler pH
pH 10,3 bis 10,7 (50 mM Glycin-NaOH-Puffer) ;
(4) optimale Temperatur
45 bis 55°C (pH 10,5, 50 mM Glycin-NaOH-Puffer) ;
(5) molekulare Masse
etwa 20,3 ± 1 kDa (gemessen durch Sedimentationsgleichgewicht; etwa 26 kDa, gemessen durch SDS PAGE):
(6) isoelektrischer Punkt
pH 9,5 bis 10,5
(isoelektrische Fokusierungs-PAGE).

2. Pectinsäurelyase gemäss Anspruch 1, die von der Gattung Bacillus abgeleitet ist.

3. Pectinsäurelyase gemäss Anspruch 1 oder 2, die von Bacillus sp. KSM-P15 (FERM BP-6241) erhältlich ist.

4. Pectinsäurelyase gemäss Anspruch 1, wobei die Deletion, der Ersatz oder die Addition von einer oder mehreren Aminosäuren durchgeführt wird, um das 107. Lys, 129. Lys und 132. Arg in der Aminosäuresequenz in SEQ ID NO: 1 zu konservieren.

5. Gen, codierend für die Pectinsäurelyase gemäss einem der Ansprüche 1 bis 4.

6. Rekombinanter Vektor, enthaltend ein Gen, wie in Anspruch 5 beschrieben.

7. Transformant, enthaltend den rekombinanten Vektor, wie in Anspruch 6 beschrieben.

8. Transformant gemäss Anspruch 7, der durch die Verwendung eines Mikroorganismus als Wirt erzeugt wird.

9. Verfahren zur Herstellung einer Pectinsäurelyase, wie in einem der Ansprüche 1 bis 3 beschrieben, **gekennzeichnet durch** Kultivieren eines Transformanten, wie in Anspruch 7 oder 8 beschrieben, und Sammeln der Pectinsäurelyase aus der Kultur.

10. Bacillus sp. KSM-P15 (FERM BP-6241).

## Revendications

1. Acide pectique lyase ayant une séquence d'acides aminés de SEQ NO 1 ou une séquence d'acides aminés ayant une homologie de 80 % ou plus à la SEQ NO 1 entre les acides aminés nos. 36-132 et ayant les propriétés suivantes :
(1) Action
de cliver par scission des liaisons α-1,4 de l'acide pectique (acide polygalacturonique) via une β-élimination d'une manière endo et de fournir une double liaison à la position C4-C5 de l'extrémité non réduite afin de former un digalacturonide insaturé ou un oligogalacturonide insaturé ;
(2) Spécificité du substrat
d'agir sur une protopectine, un acide pectique (acide polygalacturonique), un acide pectique soluble dans un acide, et une pectine ;
(3) pH optimal
pH 10,3 - 10,7 (tampon 50 mM glycine-NaOH) ;
(4) température optimale
45 - 55 °C (pH 10,5, tampon 50 mM glycine-NaOH) ;
(5) Masse moléculaire
approximativement 20,3 ± 1 kDa (comme mesurée par équilibre de la sédimentation ; approximativement 26 kDa comme mesurée par SDS PAGE) ;
(6) Point isoélectrique
pH 9,5 - 10,5 (PAGE, focalisation isoélectrique) ;

2. Acide pectique lyase selon la revendication 1, qui provient du genre Bacille.

3. Acide pectique lyase selon la revendication 1 ou 2, qui est susceptible d'être obtenue à partir du Bacillus sp. KSM-P15 (FERM BP-6241).

4. Acide pectique lyase selon la revendication 1, dans laquelle la délétion, le remplacement, ou l'addition d'un ou de plusieurs acides aminés est effectué de manière à conserver la 107^{e} Lys, la 129^{e} Lys, et la 132^{e} Arg dans la séquence d'acides aminés de la Séquence no. 1.

5. Gène codant pour l'acide pectique lyase selon l'une quelconque des revendications 1 à 4.

6. Vecteur recombinant contenant un gène comme décrit dans la revendication 5.

7. Transformant contenant le vecteur recombinant comme décrit dans la revendication 6.

8. Transformant selon la revendication 7, qui est créé par l'utilisation d'un microorganisme en tant que hôte.

9. Procédé pour la fabrication d'une acide pectique lyase comme décrite dans l'une quelconque des revendications 1 à 3, **caractérisé par** la culture d'un transformant comme décrit dans la revendication 7 ou 8 et la récupération de 11 acide pectique lyase à partir de la culture.

10. Bacillus sp. KSM-P15 (FERM BP-6241).


### SEQUENCE LISTING

<110> Kao Corporation
<120> Pectic Acid Lyase
<130> 73778 m3
<140> 98 106 586.5
   <141> 1998-04-09
<150> JP 091142/1997
   <151> 1997-04-09
<150> JP 242735/1997
   <151> 1997-09-08
<160> 32
<170> PatentIn Ver. 2.0
<210> 1
   <211> 591
   <212> DNA
   <213> Bacillus sp.
<220>
   <221> CDS
   <222> (1)..(591)
<400> 1
   gcg ccg acg gtc gtt cat gaa acg att cgt gtg cct gcc ggt cag acg   48
   ttt gac gga aaa ggg cag acc tat gtg gct aat ccg aat aca ttg ggg   96
   gac gga tcg cag gcg gag aat cag aag ccg atc ttt cgt ctg gag gct   144
   ggg gca agc ctg aaa aat gta gtg att ggc gct cct gcc gct gac ggg   192
   gtg cac tgc tac ggg gat tgt acg att aca aat gtc atc tgg gag gat   240
   gtt ggt gag gat gcg ctg acg ctt aaa tcg tcc gga acg gtg aac atc     288
   tcg ggc ggg gca gcc tac aag gcg tat gac aag gtg ttc caa atc aat     336
   gca gcg ggg acg atc aac att cgt aac ttc agg gcc gat gac atc ggg     384
   aag ctg gtt cgg cag aac gga ggc acc acc tac aaa gtg gtg atg aac     432
   gtg gaa aac tgc aac att tcc aga gtg aag gat gcg atc ctg aga acg     480
   gac agc agc aca agc aca gga cga att gtg aat acc cgc tat tct aac     528
   gtg cca aca ttg ttc aaa ggc ttt aaa tca ggc aat acc acc gca tcc     576
   gga aat acg cag tat                                                 591
<210> 2
   <211> 197
   <212> PRT
   <213> Bacillus sp.
<400> 2
<210> 3
   <211> 27
   <212> DNA
   <213> Artificial Sequence
<220>
   <223> Description of Artificial Sequence: synthetic oligonucleotide
<400> 3
   gtgtatatca aggagaagac cggcatg 27
<210> 4
   <211> 20
   <212> PRT
   <213> Bacillus sp.
<400> 4
   Ala Pro Thr Val Val His Glu Thr Ile Arg Val Pro Ala Gly Gln Thr
   1 5 10 15
   Phe Asp Gly Lys
   20
<210> 5
   <211> 12
   <212> PRT
   <213> Bacillus sp.
<400> 5
   Val Val Ile Gly Ala Pro Ala Ala Asp Gly Val His
   1 5 10
<210> 6
   <211> 26
   <212> DNA
   <213> Artificial Sequence
<220>
   <223> Description of Artificial Sequence: synthetic oligonucleotide
<400> 6
   gcnccnacng tngtncayga racnat 26
<210> 7
   <211> 23
   <212> DNA
   <213> Artificial Sequence
<400> 7
   gcngcnggng cnccdatnac nac                                                23
<210> 8
   <211> 185
   <212> DNA
   <213> Bacillus sp.
<220>
   <221> CDS
   <222> (1)..(183)
<400> 8
   gcg ccc act gtc gtg cac gaa acg att cgt gtg cct gcc ggt cag acg    48
   Ala Pro Thr Val Val His Glu Thr Ile Arg Val Pro Ala Gly Gln Thr
     1               5                  10                  15
   ttt gac gga aaa ggg cag acc tat gtg gct aat ccg aat aca ttg ggg    96
   Phe Asp Gly Lys Gly Gln Thr Tyr Val Ala Asn Pro Asn Thr Leu Gly
                20                  25                  30
   gac gga tcg cag gcg gag aat cag aag ccg atc ttt cgt ctg gag gct   144
   Asp Gly Ser Gln Ala Glu Asn Gln Lys Pro Ile Phe Arg Leu Glu Ala
            35                  40                  45
   ggg gca agc ctg aaa aat gta gtt atc ggt gca ccg gct gc            185
   Gly Ala Ser Leu Lys Asn Val Val Ile Gly Ala Pro Ala
        50                  55                  60
<210> 9
   <211> 61
   <212> PRT
   <213> Bacillus sp.
<400> 9
   Ala Pro Thr Val Val His Glu Thr Ile Arg Val Pro Ala Gly Gln Thr
     1               5                  10                  15
   Phe Asp Gly Lys Gly Gln Thr Tyr Val Ala Asn Pro Asn Thr Leu Gly
                20                  25                  30
   Asp Gly Ser Gln Ala Glu Asn Gln Lys Pro Ile Phe Arg Leu Glu Ala
            35                  40                  45
   Gly Ala Ser Leu Lys Asn Val Val Ile Gly Ala Pro Ala
        50                  55                  60
<210> 10
   <211> 26
   <212> DNA
   <213> Artificial Sequence
<220>
   <223> Description of Artificial Sequence:; synthetic oligonucleotide
<400> 10
   gcgcccactg tcgtgcacga aacgat 26
<210> 11
   <211> 23
   <212> DNA
   <213> Artificial Sequence
<220>
   <223> Description of Artificial Sequence: synthetic oligonucleotide
<400> 11
   gcagccggtg caccgataac tac 23
<210> 12
   <211> 21
   <212> DNA
   <213> Artificial Sequence
<220>
   <223> Description of Artificial Sequence: synthetic oligonucleotide
<400> 12
   caaacgtctg accggcaggc a 21
<210> 13
   <211> 25
   <212> DNA
   <213> Artificial Sequence
<220>
   <223> Description of Artificial Sequence: synthetic oligonucleotide
<400> 13
   aagccgatct ttcgtctgga ggctg 25
<210> 14
   <211> 35
   <212> DNA
   <213> Artificial Sequence
<220>
   <223> Description of Artificial Sequence: synthetic oligonucleotide
<400> 14
   gcgtcgactc gcggaggcgg cgccgacggt tgttc 35
<210> 15
   <211> 26
   <212> DNA
   <213> Artificial Sequence
<220>
   <223> Description of Artificial Sequence: synthetic oligonucleotide
<400> 15
   gcggagaatc aggcgccgat ctttcg 26
<210> 16
   <211> 27
   <212> DNA
   <213> Artificial Sequence
<220>
   <223> Description of Artificial Sequence: synthetic oligonucloeotide
<400> 16
   cggggtgcac gcctacgggg attgtac 27
<210> 17
   <211> 27
   <212> DNA
   <213> Artificial Sequence
<220>
   <223> Description of Artificial Sequence: synthetic oligonucloeotide
<400> 17 <210> 18
   <210> 18
   <211> 27
   <212> DNA
   <213> Artificial Sequence
<220>
   <223> Description of Artificial Sequence: synthetic oligonucloeotide
<400> 18
   gcgctgacgc ttgcatcgtc cggaacg 27
<210> 19
   <211> 27
   <212> DNA
   <213> Artificial Sequence
<220>
   <223> Description of Artificial Sequence: synthetic oligonucloeotide
<400> 19
   aaggcgtatg acgcggtgtt ccaaatc 27
<210> 20
   <211> 27
   <212> DNA
   <213> Artificial Sequence
<220>
   <223> Description of Artificial Sequence: synthetic oligonucloeotide
<400> 20
   gatgacatcg gggcgctggt tcggcag 27
<210> 21
   <211> 27
   <212> DNA
   <213> Artificial Sequence
<220>
   <223> Description of Artificial Sequence: synthetic oligonucloeotide
<400> 21
   gggaagctgg ttgcgcagaa cggaggc 27
<210> 22
   <211> 27
   <212> DNA
   <213> Artificial Sequence
<220>
   <223> Description of Artificial Sequence: synthetic oligonucloeotide
<400> 22
   aaggcgtatg acagagtgtt ccaaatc 27
<210> 23
   <211> 27
   <212> DNA
   <213> Artificial Sequence
<220>
   <223> Description of Artificial Sequence: synthetic oligonucloeotide
<400> 23
   aaggcgtatg accatgtgtt ccaaatc 27
<210> 24
   <211> 27
   <212> DNA
   <213> Artificial Sequence
<220>
   <223> Description of Artificial Sequence: synthetic oligonucloeotide
<400> 24
   gatgacatcg ggagactggt tcggcag 27
<210> 25
   <211> 27
   <212> DNA
   <213> Artificial Sequence
<220>
   <223> Description of Artificial Sequence: synthetic oligonucloeotide
<400> 25
   gatgacatcg ggcatctggt tcggcag 27
<210> 26
   <211> 27
   <212> DNA
   <213> Artificial Sequence
<220>
   <223> Description of Artificial Sequence: synthetic oligonucloeotide
<400> 26
   gggaagctgg ttaagcagaa cggaggc 27
<210> 27
   <211> 27
   <212> DNA
   <213> Artificial Sequence
<220>
   <223> Description of Artificial Sequence: synthetic oligonucloeotide
<400> 27
   gggaagctgg ttcaccagaa cggaggc 27
<210> 28
   <211> 24
   <212> DNA
   <213> Artificial Sequence
<220>
   <223> Description of Artificial Sequence: synthetic oligonucloeotide
<400> 28
   aatgtcatcg cggaggatgt tggt 24
<210> 29
   <211> 24
   <212> DNA
   <213> Artificial Sequence
<220>
   <223> Description of Artificial Sequence: synthetic oligonucloeotide
<400> 29
   aatgtcatct tcgaggatgt tggt 24
<210> 30
   <211> 24
   <212> DNA
   <213> Artificial Sequence
<220>
   <223> Description of Artificial Sequence: synthetic oligonucloeotide
<400> 30
   aatgtcatct acgaggatgt tggt 24
<210> 31
   <211> 36
   <212> DNA
   <213> Artificial Sequence
<220>
   <223> Description of Artificial Sequence: synthetic oligonucloeotide
<400> 31
   gcagcgggga cgatcaacat taagaacttc agggcc 36
<210> 32
   <211> 97
   <212> PRT
   <213> Bacillus sp.
<400> 32
   Gln Ala Glu Asn Gln Lys Pro Ile Phe Arg Leu Glu Ala Gly Ala Ser
    1               5                  10                  15
   Leu Lys Asn Val Val Ile Gly Ala Pro Ala Ala Asp Gly Val His Cys
               20                  25                  30
   Tyr Gly Asp Cys Thr Ile Thr Asn Val Ile Trp Glu Asp Val Gly Glu
           35                  40                  45
   Asp Ala Leu Thr Leu Lys Ser Ser Gly Thr Val Asn Ile Ser Gly Gly
       50                  55                  60
   Ala Ala Tyr Lys Ala Tyr Asp Lys Val Phe Gln Ile Asn Ala Ala Gly
   65                  70                  75                  80
   Thr Ile Asn Ile Arg Asn Phe Arg Ala Asp Asp Ile Gly Lys Leu Val
                   85                  90                  95
   Arg